# EUROPEAN PATENT APPLICATION

(11) **EP 3 210 530 A1**
(43) Date of publication of application: **30.08.2017**
(21) Application number: 16156970.2
(22) Date of filing: 23.02.2016
(51) Int. Cl.: A61B 5/024, A61B 5/00, A61B 5/0402

(54) **WRISTBAND FOR ALARMING A USER OF AN IRREGULAR HEART RHYTHM OF THE USER**

(71) Applicant: Zenicor Medical Systems AB, 113 59 Stockholm (SE)
(72) Inventor: Palerius, Mats, 113 59 Stockholm (SE); Norström, Sonny, 113 59 Stockholm (SE); Öhlund, Emma, 113 59 Stockholm (SE)
(74) Representative: Awapatent AB

(57) **Abstract**

A wristband intended for alarming a user wearing the wristband of an irregular heart rhythm of the user is provided. The wristband comprises a sensor unit arranged to measure a signal pertaining to the heart rhythm of the user and a clock unit arranged to provide a time frame, a signal unit arranged to derive a time-resolved signal and a data storage unit arranged to store data. The wristband further comprises a power unit and an alarm unit. The wristband further comprises a frequency for calculating the frequency of the time-resolved signal, an amplitude unit for calculating the amplitude of said time-resolved signal and an analyzing unit arranged to perform a frequency analysis and an amplitude analysis and to perform the frequency analysis and the amplitude analysis simultaneously or in parallel and to send an alarm command to the alarm unit for alarming the user of an irregular heart rhythm.

## Description

The present disclosure relates to medical devices and methods. In particular, the present disclosure relates to devices and methods for alarming a user of an irregular heart rhythm of the user.

### Background art

Cardiovascular diseases are the leading cause of death in the world. In 2008, 30% of all global death can be attributed to cardiovascular diseases. It is also estimated that by 2030, over 23 million people will die from cardiovascular diseases annually. Cardiovascular diseases are prevalent in the populations of high-income and low-income countries alike.

Irregular heart rhythm is a cardiac condition in which the electrical activity of the heart is irregular or is faster or slower than normal. Although not always life-threatening, some conditions can cause cardiac arrest and even sudden cardiac death. Atrial fibrillation is the most common form of irregular heart rhythm. In atrial fibrillation, electrical conduction through the ventricles of heart is irregular and disorganized. While atrial fibrillation may cause no symptoms, it is sometimes associated with palpitations, shortness of breath, fainting, chest, pain or congestive heart failure. Atrial fibrillation is also associated with atrial clot formation, which is associated with clot migration and stroke.

Atrial fibrillation is typically diagnosed by taking an electrocardiogram (ECG) of a subject, which shows a characteristic atrial fibrillation waveform. An inherent challenge is that, for many subjects, irregularities in the heart rhythm do not occur that frequently and causes no symptoms. Hence, an ECG taken at a hospital or other health care facility may have a high likelihood of missing out on important information of the medical condition of the subject, just because the subject's heart behaved well during the ECG session. To alleviate this problem, ECG measurements can be carried out over a longer period of time. Typically, the subject will self-administer ECG measurements at home during a predetermined period of time, for example 2-3 weeks. The subject is equipped with a portable ECG device, which is arranged to collect data of the subject's heart condition. One such ECG device is the Zenicor-ECG. The Zenicor-ECG is a handheld ECG device, known as a thumb ECG, with which the subjects register their ECG data themselves by placing their thumbs on two electrodes for 30 seconds. By pressing a button the readings are transferred via the mobile network to a central ECG database.

A particular problem with the ECG measurement periods discussed here is that, though convenient and straight-forward to use, the measurements are ongoing for a relatively short period of time, such as e.g. 30 seconds for the Zenicor-ECG. As many irregularities cause no symptoms and may occur unnoticed by the subject, it is difficult to actively choose when to take an ECG. Instead one relies on increasing the total time duration of data collection. However, even if a subject uses the device regularly over several weeks of time, the likelihood of missing important heart irregularities remain.

### Summary

It is an object to mitigate, alleviate or eliminate one or more of the above-identified deficiencies in the art and disadvantages singly or in any combination and solve at least the above mentioned problem.

According to a first aspect, these and other objects are achieved in full, or at least in part, by a wristband intended for alarming a user wearing the wristband of an irregular heart rhythm of the user. The wristband comprises a sensor unit arranged to measure a signal pertaining to the heart rhythm of the user. The wristband further comprises a clock unit arranged to provide a time frame, a signal unit arranged to derive a time-resolved signal and a data storage unit arranged to store data. The wristband further comprises a power unit and an alarm unit. The wristband further comprises a frequency unit arranged to calculate the frequency of the time-resolved signal, an amplitude unit arranged to calculate the amplitude of said time-resolved signal and an analyzing unit arranged to perform a frequency analysis and an amplitude analysis. The analyzing unit is further arranged to perform the frequency analysis and the amplitude analysis simultaneously or in parallel. The analyzing unit is further arranged to send an alarm command to the alarm unit, pertaining to alarming the user of an irregular heart rhythm.

The wristband is advantageous as it allows online monitoring of the heart rhythm of the user during long periods of time, such as e.g. days or weeks. The purpose of alarming the user is to aid the user in deciding when to apply a second device to measure an ECG. Such a second device may be for example a Zenicor-ECG. Another advantage is that the wristband comprises all units needed for measuring a signal pertaining to the heart rhythm, analysing the signal and alarming the user if irregular heart rhythm has been detected.

According to one embodiment, the wristband further comprises a second sensor unit arranged to measure the g-force of the user. This may be advantageous as it allows the wristband to take the movement of the user into account during analysis of the signal pertaining to the heart rhythm of the user.

According to one embodiment, the sensor unit is arranged to measure a signal pertaining to the blood oxygen level of the user optically using two measurement systems operating at separate wavelengths. This may be advantageous at it allows for a non-invasive accurate and fast online monitoring of the heart rhythm of the user.

According to one embodiment, the alarm unit comprises a tactile unit arranged to induce a tactile sensation in the user pertaining to informing the user that it is time to take an ECG using a second device. This may be advantageous as is allows for a fast and direct alarm by minimizing the time between the identification of an irregular heart rhythm of the user and the start of ECG measurement using a second device.

According to one embodiment, the alarm unit comprises a speaker unit arranged to emit a sound alarm pertaining to informing the user that it is time to take an ECG using a second device. This may be advantageous as is allows for additional warning signals intended for the user to react to the wristband. Additionally, the sound alarm may be used to give instructions to the user comprising more details, such as e.g. the number of irregularities, or the type of irregularities.

According to one embodiment, the alarm unit comprises a light-emitting unit arranged to emit a visual alarm pertaining to informing the user that it is time to take an ECG using a second device. This may be advantageous as is allows for yet another additional warning signal intended for the user to react to the wristband. Likewise, the visual alarm may be used to give instructions to the user comprising more details, such as e.g. the number of irregularities, or the type of irregularities.

According to one embodiment, all units of the wristband are contained in a casing. This may be advantageous as is allows for the wristband and its operations to be independent of any other device such as e.g. mobile phones, servers, computers etc. Additionally, the wristband is may be essentially maintenance free and easy to use.

According to a second aspect a method is provided. The method is intended for alarming a user of an irregular heart rhythm of the user. The method comprises a number of steps. In a first step a sensor unit measures a signal pertaining to the heart rhythm of the user. In a next step a signal unit creates a time-resolved signal using input from the sensor unit and input from a clock unit. In a next step a frequency unit calculates the frequency of the time-resolved signal. In yet another step an amplitude unit calculates the amplitude of the time-resolved signal. In yet another step an analyzing unit analyses the frequency and the amplitude. The analysis of the frequency comprises monitoring for a frequency irregularity and the analysis of the amplitude comprises monitoring for an amplitude irregularity. In yet another step the analyzing unit determines if a frequency irregularity and an amplitude irregularity occurs within a time interval. In case at least a frequency irregularity or an amplitude irregularity occurs within a time interval, the analyzing unit sends an alarm signal to the alarm unit. In a next step the alarm unit alarms the user of an irregular heart rhythm.

The method may be advantageous as it allows for a fast and reliable monitoring of the heart rhythm of the user. It further allows for a fast and reliable identification of an irregular heart rhythm of the user. It further allows for alarming the user, thus fulfilling the purpose of letting the user know that it is time to take an ECG with a second device. Such a second device may be for example a Zenicor-ECG.

According to one embodiment, the step of measuring, using a sensor unit, the signal pertaining to the heart rhythm of the user further comprises measuring, using a second sensor unit, a second signal pertaining to the g-force of the user. The alarm unit is disabled from alarming the user if said second signal exceeds a threshold value. This may be advantageous as it allows taking the movement of the user into account during analysis of the signal. This, in turn, allows for the sensor unit to be able to measure the signal during time periods where signal quality is optimal. Moreover, misreading due to high degree of movement of the user may be avoided.

According to one embodiment, the sensitivity for when alarming a user of an irregular heart rhythm of the user is adjustable by means of adjusting a frequency irregularity threshold value, C_{f}, and an amplitude irregularity threshold value, C_{A}. The threshold values are input from a user. This may be advantageous as it allows for adjusting the wristband to provide optimal performance for each user.

According to one embodiment, the sensitivity for when alarming a user of an irregular heart rhythm of the user is adjustable by means of adjusting a tolerance number. The tolerance number, which is available to the analyzing unit, is used by the analyzing unit to determine the number of times the frequency irregularity and the amplitude irregularity occurring within the time interval is allowed to occur within a second time interval, before the alarm unit is disabled from alarming the user. The second time interval is longer than the first time interval. The tolerance number is input from a user. This may be advantageous as it allows for adjusting the wristband to provide optimal performance for each user.

According to one embodiment, the step of calculating, by a frequency unit, the frequency of a part of said time-resolved signal comprises calculating a series of frequency values, each frequency value within the series of frequency values being the inverse of the time period between two adjacent peaks in the time-resolved signal. Furthermore, the step of calculating, by an amplitude unit, the amplitude of a part of said time-resolved signal comprises calculating a series of amplitude values, each amplitude value in the series of amplitude values being derived from a single peak in the time-resolved signal. The step of analysing, by an analyzing unit, the frequency and the amplitude comprises comparing at least one of the frequency values with one or more other frequency values and comparing at least one of the amplitude values with one or more other amplitude values. This may be advantageous as it allows for taking into account not only the most recently recorded time-resolved signal, but also previously recorded parts of the time-resolved signal. It further allows for a statistical analysis based on more data which also may cover a longer time period. This, in turn, may increase the accuracy of the alarm function, thus avoiding alarming the user of regularly occurring irregularities which may be of less clinical importance for some users. It further allows for a more robust analysis less prone to measurement error.

According to one embodiment, the step of analysing, by an analyzing unit, the frequency and the amplitude comprise nonlinear methods comprising a Poincare plot. The analysis is carried out using at least one standard deviation calculated along at least one direction within the plane defined by the Poincare plot.

According to one embodiment, the alarm unit induces a tactile sensation in the user. The tactile sensation pertains to inform the user that it is time to take an ECG using a second device.

According to one embodiment, the alarm unit emits one or more from the list of a sound alarm pertaining to inform the user that it is time to take an ECG using a second device, a visual alarm pertaining to inform the user that it is time to take an ECG using a second device.

Please note that the embodiments above can be combined within the scope of the appended claims unless such combinations are clearly contradictory.

### Brief description of the drawings

The embodiments will by way of example be described in more detail with reference to the appended schematic drawings, which shows presently preferred embodiments.
Figure 1 is a schematic view of the wristband according to one embodiment.
Figure 2 is a schematic view of an example of a time-resolved signal forming the basis for detecting an irregular heart rhythm of a user.
Figure 3 is a schematic view of a method according to one embodiment.
Figure 4 is a schematic view of an example of a Poincare plot according to one example embodiment.
Figure 5 is a schematic view of an example of a time-resolved signal forming the basis for detecting an irregular heart rhythm of a user according to one example embodiment.

### Detailed description

In the following a detailed description of embodiments will be given with reference to the accompanying drawings. It will be appreciated that the drawings are for illustration only and are not in any way restricting the scope of the claims. Thus, any references to directions, such as "up" or "down", are only referring to the directions shown in the figures. It should be noted that the features having the same reference numerals have the same function, a feature in one embodiment could thus be exchanged for a feature from another embodiment having the same reference numeral, unless clearly contradictory. The descriptions of the features having the same reference numerals should thus be seen as complementing each other in describing the fundamental idea of the feature and thereby showing the features versatility.

In the description and the claims, the following terminology will be used in accordance with the definitions set forth below. Unless defined otherwise, all technical and scientific terms used herein have the same meaning as commonly understood by one of ordinary skill in the art.

It should be understood that the **frequency** referred to in this disclosure is to be based on the time period between two consecutive pulses in a pulse train occurring in the time-resolved signal, said pulses being correlated to the heart rhythm. The frequency may be one or more instantaneous values (i.e. determined by the inverse of the time between two consecutive pulses) but may also refer to one or more average frequencies, averaged over any number of pulses.

It should be understood that the **amplitude** referred to in this disclosure is based on the signal peaks occurring in the time-resolved signal, said signal peaks being correlated to the heart rhythm. Said amplitude may be based on calculating one or more maximum values at one or more signal peaks. It may or may not involve withdrawing a background level. Alternatively, the amplitude could be based on integrating a part of the signal during at least part of a peak. The amplitude may be one or more instantaneous values (i.e. be based on calculations at individual peaks) but may also refer to an average amplitude, averaged over any number of pulses.

A wristband is provided with the purpose of alarming a user of the wristband that the user has an irregular heart rhythm. The purpose of alarming the user is to aid the user in deciding when to apply a second device to measure an ECG. Such a second device may be for example a Zenicor-ECG. Alternatively, it may be a device of another brand or a device of another type, measuring the ECG of the user. Thus, the wristband is not intended for monitoring the health of the user. It acts merely as a trigger unit for initiating measurements with the second device. The wristband is optimized for its task, and therefore focuses on fast and accurate determination of when to issue an alarm. The wristband is intended to be worn at home during a period of days or weeks. Thus the wristband is intended to be essentially maintenance free and easy to use. The wristband is arranged to measure a signal pertaining to the heart rhythm of the user. Such a signal could be for example an optical signal. This is advantageous as it enables non-invasive online monitoring at the wrist without requirements of additional data/sensors. The signal measured by the wristband will be analysed in the wristband, said analysis being optimized for speed and accuracy for the task of determining irregularities in the heart rhythm.

The analysis comprises detecting deviations from expected values in the signal, said deviations being detected in the signal frequency and the signal amplitude. In a case of heart beat irregularity, the wristband will detect a deviation in the frequency of the signal with respect to an expected frequency. The wristband will also detect a deviation in the amplitude of the signal with respect to an expected amplitude. In case these deviations are considered to be sufficiently high, and occurring sufficiently close to each other in time, the wristband will alarm the user of a detected irregularity in the heart rhythm.

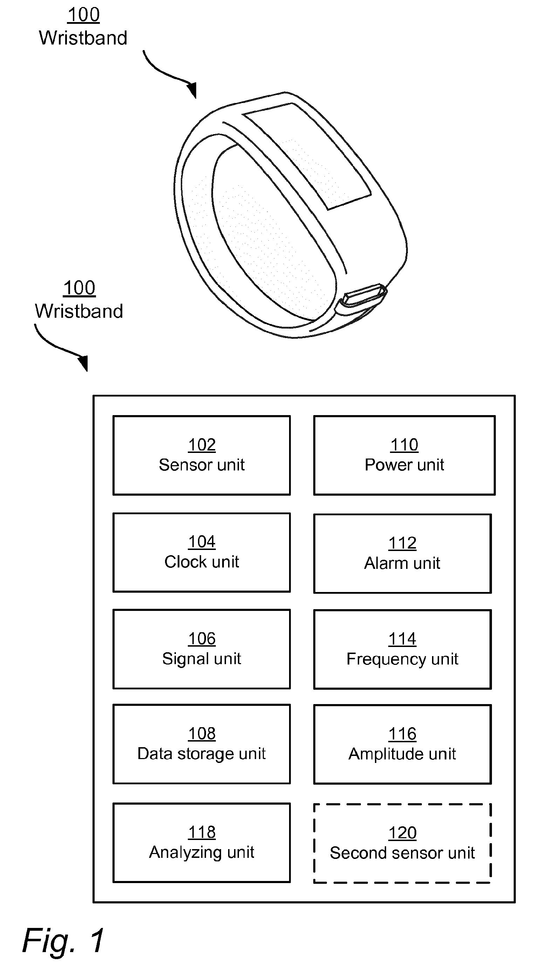

Figure 1 illustrates a wristband 100 for alarming a user wearing said wristband of an irregular heart rhythm of the user according to one embodiment. The wristband 100 comprises a sensor unit 102 arranged to measure a signal pertaining to the heart rhythm of the user. The sensor unit 102 may comprise an optical sensor. Such an optical sensor may for example be arranged to measure in transmissive or reflective application mode. Such an optical sensor may be of a type typically used for pulse oxymetry. The sensor unit may comprise more than one optical sensor. In one example embodiment, two optical sensors are used to measure simultaneously at two different wavelengths. In one example embodiment, the signal is correlated to the blood oxygen level of the user. Alternatively, the sensor unit 102 may comprise a sensor of any other type capable of measuring a signal pertaining to the heart rhythm of the user.

The wristband 100 further comprises a clock unit 104 arranged to provide a time frame. The time frame is needed for signal processing purposes as will be described later. Said clock unit 104 may comprise for example a crystal oscillator circuit.

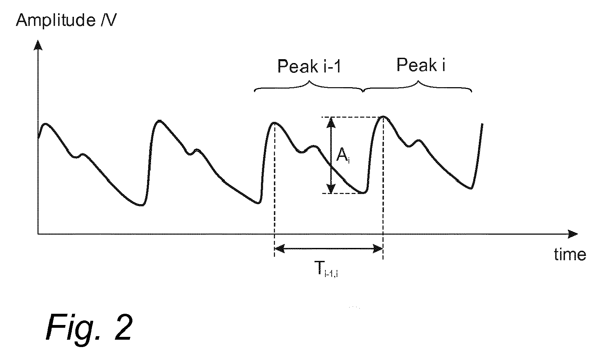

The wristband 100 further comprises a signal unit 106 arranged to derive a time-resolved signal. An example of such a time-resolved signal is shown in Fig. 2. The time-resolved signal is derived by using input from the sensor unit 102 and input from the clock unit 104. The time-resolved signal will thus represent the heart rhythm of the user as function of time. The time-resolved signal may be for example a photoplethysmogram (PPG). The time-resolved signal may be digitized by the signal unit 106, but may, alternatively, be digitized by an alternative unit part of the wristband 100. The digitization may be carried out using different time resolution, i.e. number of samples per second.

The wristband 100 further comprises a data storage unit 108 arranged to store data. The data storage unit may for example comprise a buffer such as for example a solid state storage unit. This is advantageous as it permits storing and retrieving data such as for example the time-resolved signal pertaining to the heart rhythm of the user, but also other data such as for example settings and instructions for the operation of the wristband 100, log files pertaining to the operation of the wristband, statistics regarding the detected heart rhythm irregularities etc.

The wristband 100 further comprises a power unit 110 arranged to supply power to one or more of the units of the wristband 100. The power unit 110 may for example comprise a battery, either rechargeable or not rechargeable. Said battery may be removable but may also be built into the wristband 100.

The wristband 100 further comprises an alarm unit 112. The alarm unit 112 may comprise a tactile unit arranged to induce a tactile sensation in the user. The alarm unit 112 may alternatively comprise a speaker unit arranged to emit a sound alarm. The alarm unit 112 may alternatively comprise a light-emitting unit arranged to emit a visual alarm. The light-emitting unit may comprise a light-emitting diode LED or any other types of light-emitting components. Specifically, it may comprise a display unit arranged to visually alarm the user. The purpose of the alarm unit is to inform the user of the wristband 100 that it is time to take an ECG using a second device. Such a second device may be for example a Zenicor-ECG. Alternatively, it may be a device of another brand or a device of another type, measuring the ECG of the user. The alarm unit 112 may provide additional details to the user, such as e.g. the level of importance of an alarm, the relative amount of energy available in the power unit 110 etc. The purpose of the alarm unit 112 is to alarm the user when the wristband has detected an irregular heart rhythm of the user. The user will then be able to take action accordingly, the intention being to take an ECG with a second device.

The wristband 100 further comprises a frequency unit 114 arranged to calculate the frequency of said time-resolved signal. The frequency unit 114 may calculate the frequency in many different ways, and, as will be detailed later, the frequency unit 114 may output frequency data based on calculations of instantaneous frequencies but may also output frequency data based on calculations of time averages.

The wristband 100 further comprises an amplitude unit 116 arranged to calculate the amplitude of said time-resolved signal. The amplitude unit 116 may calculate the amplitude in many different ways, and, as will be detailed later, the amplitude unit 116 may output amplitude data based on calculations of instantaneous amplitudes but may also output amplitude data based on calculations of time averages.

The wristband 100 further comprises an analyzing unit 118 arranged to perform an analysis of said frequency and an analysis of said amplitude. For this purpose, the analysis unit 118 is supplied with data output from the frequency unit 114 and the amplitude unit 116. The analyzing unit 118 is further arranged to perform said frequency analysis and said amplitude analysis simultaneously or in parallel. The analyzing unit 118 is further arranged to send an alarm command to the alarm unit 112, pertaining to alarming the user of an irregular heart rhythm. The role of the analyzing unit 118 is to take a decision whether or not an irregularity in the heart rhythm of the user has occurred. It is aided in this task by supply of input data from other units, such as the frequency unit 114 and the amplitude unit 116, but the analyzing unit also utilizes input data from a user, as will be detailed later.

In an alternative example embodiment, the wristband 100 further comprises a second sensor 120 unit arranged to measure the g-force of the user. Such a sensor unit may comprise for example an accelerometer. Such an accelerometer may comprise for example piezoelectric, piezoresistive and capacitive components. The role of the second sensor 120 is to supply the analyzing unit with additional data pertaining to the movement of the user. This may optimize the collection of data from the sensor unit 102, and, additionally, increase the accuracy of the heart rhythm irregularity detection.

The wristband 100 may be arranged to perform all operations needed for its purpose without use of units outside of the wristband. Preferably, the wristband thus comprises all units disclosed herein within a casing.

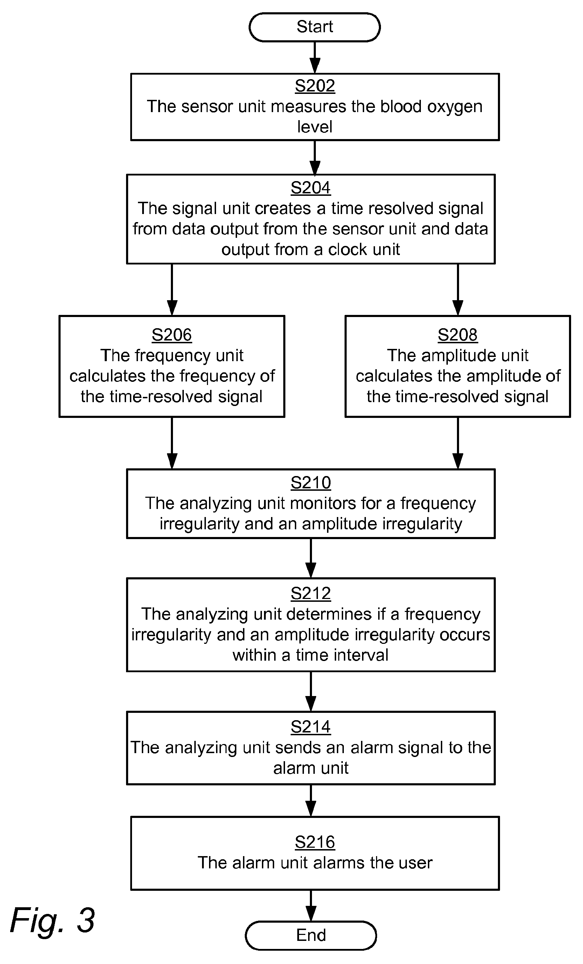

A series of method steps in the wristband 100 will now be described with reference to the Fig. 3, said method steps being attributed to alarming a user of an irregular heart rhythm of the user. In a first step S202, the sensor unit 102 is used to measure a signal pertaining to the heart rhythm of the user. The sensor unit 102 outputs measurement data to the signal unit 106. The clock unit 104 outputs a time reference to the signal unit 106. The signal unit 106 creates a time-resolved signal S204 using input from the sensor unit 102 and input from the clock unit 104. The time-resolved signal may comprise more than one signal trace. Specifically, in one embodiment, the time-resolved signal comprises two signal traces, one for each of the two separate wavelengths utilized for measurements in the sensor unit. Each one of the one or more signal traces is representing a measured signal as function of time. The role of the signal unit is to prepare the data from the sensor unit 102 in a format accessible by other units in the wristband. This comprises incorporating time information in the signal, but may also comprise preparing the data from the sensor unit 102 in various ways, such as for example, digitizing, resampling, subtracting a background, filtering, smoothing, etc.

The frequency unit 114 calculates the frequency of the time-resolved signal S206. The frequency unit 114 may calculate the frequency in many different ways. For example, the frequency unit 114 may calculate an instantaneous frequency value pertaining to the present moment in time, for example by comparing the time position of the two peaks representing the most recently recorded maxima in the signal. An example of how this may be realised is given in Fig. 2, showing a time-resolved signal with the latest recorded five periods. The instantaneous frequency value pertaining to the present moment in time is, in this example, estimated by determining the time period, T_{i-1,i}, between the most recently recorded peak, peak i, and the peak prior to that, i-1. The frequency value pertaining to the present moment in time may then be calculated as f_{i-1,i}=1/T_{i-1,i}. Alternatively, the frequency unit 114 may calculate average frequencies of the time-resolved signal over a time period comprising more than two Such time periods could be relatively short, covering time periods close to the latest recorded part of the time-resolved signal. Time periods may also be longer, covering e.g. seconds or minutes of the time-resolved signal. Time periods may or may not comprise the most recently recorded part of the time-resolved signal. The averaging may be carried out using arithmetic mean calculations but may alternatively be carried out using another formulation, such as for example the median. The averaging may be directly performed on a group on instantaneous frequency values, but should not be limited to that. For example the averaging could be performed on a group of average frequency values, where each average frequency value is based on a calculation of the average over instantaneous frequency values. The role of the frequency unit is to supply, to the analyzing signal, data on the frequency of the time-resolved signal. This comprises determining a frequency essentially pertaining to the present moment in time, i.e. at least based on the two most recently created peaks in the time-resolved signal. The frequency unit is, however, also responsible for preparing one or more sets of data to be used as reference frequency data. Such reference frequency data could be for example the average frequency values disclosed herein.

The amplitude unit 116 calculates the amplitude of the time-resolved signal S208. The amplitude unit 116 may calculate the amplitude in many different ways. For example, the amplitude unit 116 may calculate the instantaneous amplitude value pertaining to the present moment in time, for example by determining the maximum and minimum values of the time-resolved signal. An example of how this may be realised is given in Fig. 2, where the instantaneous amplitude value pertaining to the present moment in time is estimated using the difference, Aᵢ, between the maximum amplitude of the latest recorded peak, peak i, and the minimum amplitude measured just prior to said peak i. Alternatively, the amplitude unit 114 may calculate averages of instantaneous amplitude values inferred from the time-resolved signal over a time period comprising more than one peak. Such time periods could be relatively short, covering time periods close to the latest recorded part of the time-resolved signal. Time periods may also be longer, covering e.g. seconds or minutes of the time-resolved signal. Time periods may or may not comprise the lasts part of the time-resolved signal. The averaging may be carried out using arithmetic mean calculations but may alternatively be carried out using another formulation, such as for example a calculation of the median. The averaging may be directly performed on a group on instantaneous amplitude values, but should not be limited to that. For example the averaging could be performed on a group of average amplitude values, where each average amplitude value is based on a calculation of the average over instantaneous amplitude values. The role of the amplitude unit is to supply, to the analyzing signal, data on the amplitude of the time-resolved signal. This comprises determining an amplitude essentially pertaining to the present moment in time, i.e. at least based on the amplitude of the most recently created peak in the time-resolved signal. The amplitude unit is, however, also responsible for preparing one or more sets of data to be used as reference amplitude data. Such reference amplitude data could be for example the average amplitude values disclosed herein.

The analyzing unit 118 analyses the frequency and the amplitude S210. The analysis of the frequency comprises monitoring for a frequency irregularity and the analysis of the amplitude comprises monitoring for an amplitude irregularity. The analyzing unit may distinguish frequency irregularities from non-irregularities in frequency by comparing two or more frequency values input from the frequency unit to the analyzing unit. The analyzing unit may further distinguish amplitude irregularities from non-irregularities in amplitude by comparing two or more amplitude values input from the amplitude unit to the analyzing unit. The analyzing unit may make a decision based upon detected irregularities in only one of said quantities. The analyzing unit may, alternatively, make a decision based upon irregularities to have been detected in both the frequency and the amplitude of the time resolved signal.

A frequency irregularity may comprise a deviation between a frequency value and an expected frequency value. Likewise, an amplitude irregularity may comprise a deviation between an amplitude value and an expected amplitude value. An expected frequency value may for example be an average over several peaks in the time-resolved signal. Similarly, an expected amplitude value may for example be an average over several peaks in the time-resolved signal. It may be preferable to allow a tolerance within which the deviations in frequency and amplitude are allowed to vary without alarming a user. The sensitivity determining the minimum deviation required for the analysing unit to detect a frequency irregularity can be adjustable by means of adjusting a frequency irregularity threshold value, C_{f}. Similarly, the sensitivity determining the minimum deviation required for the analysing unit to detect an amplitude irregularity can be adjustable by means of adjusting an amplitude irregularity threshold value, C_{A}. The threshold values C_{f} and C_{A} may be input from a user.

One prerequisite for the analysing unit to acknowledge an irregularity in the heart rhythm of the user is that irregularities are detected in both the frequency and the amplitude within a time interval S212. This means that, as an example, if the analyzing unit detect an amplitude irregularity during the most recently recorded 5 seconds of the time resolved signal, but the analyzing unit does not detect a corresponding frequency irregularity during the same 5 seconds, the analyzing unit will not consider the event to be an irregularity in the heart rate of the user.

For some users, the heart rhythm may show frequently occurring irregularities of less clinical importance. To prevent the wristband 100 from alarming excessively, the sensitivity of the wristband for when alarming a user of an irregular heart rhythm of the user may also adjustable by means of adjusting a tolerance number, available to the analyzing unit 118, for said analyzing unit 118 to determine the number of times said frequency irregularity and said amplitude irregularity occurring within said time interval is allowed to occur within a second time interval, before said alarm unit 112 is disabled from alarming the user, said second time interval being longer than said first time interval, wherein said tolerance number is being input from a user.

Thus, by adjusting the frequency irregularity threshold value, C_{f}, the amplitude irregularity threshold value, C_{f}, and the tolerance number, the wristband may be adjusted for its operation to be optimal for the user.

If the criterions outlined above are met, the final step of the analyzing unit 118 is to send an alarm signal to the alarm unit 112, S214. The alarm unit 112 will then alarm the user of an irregular heart rhythm S216. The alarm unit will be discussed in more detail later.

A second sensor 120 unit may be used to measure a signal pertaining to the g-force of the user. The second sensor 120 unit may comprise for example an accelerometer. The signal from the second sensor unit 120 may be used to disable the alarming of the wristband. This may provide more reliable operation of the wristband as it enables focusing on time periods where the user is relatively calm. This may be advantageous for several reasons. One reason is that the measurements carried out by the sensor unit 102 is expected to show better quality of the signal when measurement are carried out during period of a user being calm, as the wristband is less likely to move, thus risking introducing errors. Another reason is that the ECG that is intended to be taken just after a detection of an irregular heart Rhythm is to be taken when the user is calm. The disabling of the alarm may be implemented in many ways. For example, the analyzing unit may be arranged to ignore analysing the time-resolved signals at time periods where the signal from the second sensor unit 120 exceeds a threshold value, said threshold value being correlated to a certain amount of movement of the user. Alternatively, the alarm unit may be arranged to not send an alarm unless the signal from the second sensor unit 120 exceeds a threshold value.

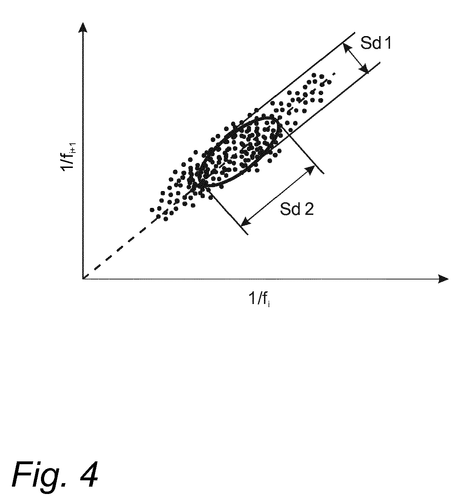

Here will be provided an example of how the wristband 100 may discern an irregular heart rhythm of a user. The frequency unit and the amplitude unit output frequency data and amplitude data. The data is in this example, preferentially, momentary values, i.e. the frequency being the inverse of the time period between the two adjacent peaks and the amplitude being the total rise of the signal magnitude of one peak as detailed in Fig 2. The analyzing unit receives the frequency data and the amplitude data for a predetermined time period and performs an analysis pertaining to indentifying an irregular heart rhythm of the user. Such an analysis may be for example a recurrence plot. In the example, the statistical analysis is a Poincare plot. One Poincare plot is derived for the frequency and one for the amplitude. An example of a Poincare plot for the frequency is shown in Fig. 4. In the Poincare plot shown in Fig. 4, each value of the inverse of the frequency from pulse i is plotted as function of the reciprocal of the frequency for the pulse prior to that, i.e. pulse i-1. This process continues recursively for the whole predefined time period. A scatter plot is created and an analysis of the data may comprise many methods. One example of such a method is to calculate the total standard deviation, SD1, pertaining to the short-term pulse-to-pulse variations and the total standard deviation, SD2, pertaining to the long-term interval variability. SD1 will then be graphically represented by a line perpendicular to the general direction of the elongated form of the scatter plot. SD2 will be graphically represented by a line parallel with the direction of the elongated form of the scatter plot. The analyzing unit receives an irregularity threshold value defining allowed regions for SD1 and SD2, and is arranged to alarm the user of an irregular heart rhythm of the user if the results from the analysis, i.e. SD1 and SD2 occurs outside of the allowed region. Such an allowed region could be for example an ellipse (see Fig. 4), said ellipse having its long radius equal to SD2 and its short radius equal to SD1. The amplitude may be analysed following the same methodology, creating a Poincare plot with amplitude values.

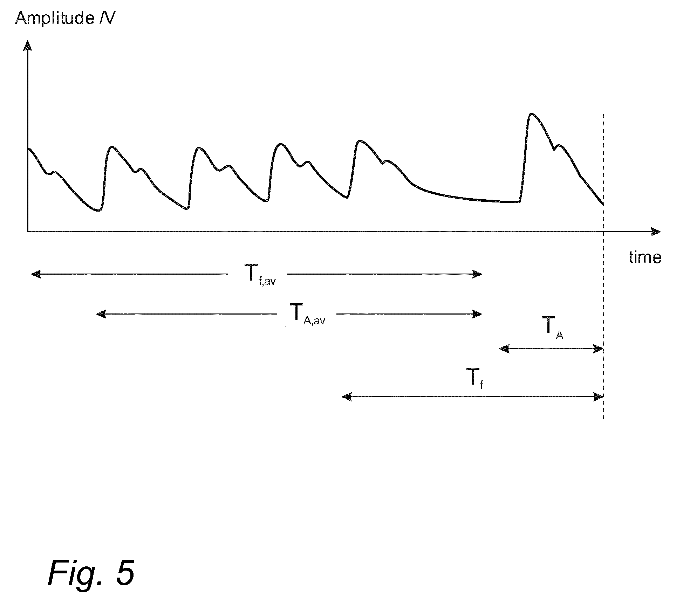

Here will be provided another example of how the wristband 100 may discern an irregular heart rhythm of a user. The example will be discussed with reference to Fig. 5. The sensor unit provides measurement data pertaining to the heart rhythm of the user, and, as has been detailed above, the frequency unit and the amplitude unit will be provided with a time-resolved signal comprising at least one time-resolved trace pertaining to the heart rhythm of the user.

In this example, the frequency unit 114 calculates the frequency of the time-resolved signal in two ways. A first average signal frequency, fₐᵥ, is calculated over the time duration T_{fav}. A second average signal frequency, f, is calculating, over the time duration T_{f}. The averaging procedure is following the procedure outlined earlier with reference to Fig. 2. It should be noted that T_{f} must be long enough to cover at least two peaks in the time-resolved signal, but could alternatively cover more than two. T_{f,av} must always be longer than T_{f}.

Similarly, the amplitude unit 116 calculates the amplitude of the time-resolved signal in two ways. A first average signal amplitude, Aₐᵥ, is calculated over the time duration T_{A,av}. A second average signal amplitude, A, is calculating, over the time duration T_{A}.. The averaging procedure is following the procedure outlined earlier with reference to Fig. 2. It should be noted that T_{A} must be long enough to cover at least one peak in the time-resolved signal, but could alternatively cover more than one. T_{A,av} must always be longer than T_{A}.

It is important that T_{f} and T_{A} have a mutual overlap in time and that T_{f} and T_{A} comprises the most recently created part of the time-resolved signal. This ensures that the wristband is capable of alarming as soon as possible thus minimizing delaying to the analysis procedure.

The analyzing unit 118 then analyses said frequency and said amplitude by deriving the quantities |*f-fₐᵥ*| and *A - Aₐᵥ* and investigating if |*f - fₐᵥ*| > *C_{f}* and *A - Aₐᵥ > C_{A}.*

Alternatively, many additional methodologies may be carried out instead of, or in combination with the methods disclosed herein, on the frequency and amplitude data within the scope of the present disclosure. Such methods are well known in the art and comprise for example recurrence quantification analysis, power frequency analysis etc.

If the analyzing unit acknowledges an irregular heart rhythm, the analyzing unit will send a command to the alarm unit, whereby the alarm unit will alarm the user. This will be realised by the alarm unit inducing a tactile sensation in the user. The tactile sensation may be induced by way of e.g. a vibration. The alarm unit may additionally alarm the user using a sound alarm. This may be for example a beeping sound, or a melody. The alarm unit may additionally alarm the user using a visual alarm. This may be for example a flashing LED, or any other kind of light-emitting component. It may also comprise a display unit, capable of informing the user of details regarding the alarm. These details may be for example, the number of detected irregularities, the number of alarms given for a time period, the condition of the wristband etc.

## Claims

1. A wristband (100) for alarming a user wearing said wristband of an irregular heart rhythm of the user, the wristband (100) comprising:
a sensor unit (102) arranged to measure a signal pertaining to the heart rhythm of the user,
a clock unit (104) arranged to provide a time frame,
a signal unit (106) arranged to derive a time-resolved signal,
a data storage unit (108) arranged to store data,
a power unit (110),
an alarm unit (112),
a frequency unit (114) arranged to calculate the frequency of said time-resolved signal,
an amplitude unit (116) arranged to calculate the amplitude of said time-resolved signal,
an analyzing unit (118) arranged to perform a frequency analysis and an amplitude analysis, wherein said analyzing unit (118) is further arranged to perform said frequency analysis and said amplitude analysis simultaneously or in parallel, and wherein
said analyzing unit (118) is further arranged to send an alarm command to the alarm unit (112), pertaining to alarming the user of an irregular heart rhythm.

2. The wristband (100) according to claim 1, further comprising a second sensor unit (120) arranged to measure the g-force of the user.

3. The wristband (100) according to claim 1-2, wherein the sensor unit is arranged to measure a signal pertaining to the blood oxygen level of the user optically using two measurement systems operating at separate wavelengths.

4. The wristband (100) according to claim 1-3 wherein the alarm unit (112) comprises a tactile unit arranged to induce a tactile sensation in the user pertaining to informing the user that it is time to take an ECG using a second device.

5. The wristband (100) according to claim 1-4 wherein the alarm unit (112) comprises a speaker unit arranged to emit a sound alarm pertaining to informing the user that it is time to take an ECG using a second device.

6. The wristband according to claim 1-5 wherein the alarm unit comprises a light-emitting unit arranged to emit a visual alarm pertaining to informing the user that it is time to take an ECG using a second device.

7. The wristband according to claim 1-6 wherein all units are contained in a casing.

8. A method for alarming a user of an irregular heart rhythm of the user, the method comprising the steps of:
a) measuring (S202), using a sensor unit (102), a signal pertaining to the heart rhythm of the user,
b) creating (S204), using a signal unit (106), a time-resolved signal using input from said sensor unit (102) and input from a clock unit (104),
c) calculating (S206), by a frequency unit (114), the frequency of said time-resolved signal,
d) calculating (S208), by an amplitude unit (116), the amplitude of said time-resolved signal,
e) analysing (S210), by an analyzing unit (118), said frequency and said amplitude, wherein said analysis of said frequency comprises monitoring for a frequency irregularity and said analysis of said amplitude comprising monitoring for an amplitude irregularity,
f) determining (S212), by the analyzing unit (118), if a frequency irregularity and an amplitude irregularity occurs within a time interval,
g) sending (S214), in case at least a frequency irregularity or an amplitude irregularity occurs within a time interval, by the analyzing unit (118), an alarm signal to the alarm unit (112),
h) alarming (S216), by the alarm unit (112), the user of an irregular heart rhythm.

9. The method according to claim 8, wherein the step of measuring (S202), using a sensor unit (102), the signal pertaining to the heart rhythm of the user further comprises measuring, using a second sensor unit (120), a second signal pertaining to the g-force of the user, and wherein said alarm unit (112) is disabled from alarming the user if said second signal exceeds a threshold value.

10. The method according to claim 8-9, wherein the sensitivity for when alarming a user of an irregular heart rhythm of the user is adjustable by means of adjusting a frequency irregularity threshold value, C_{f}, and an amplitude irregularity threshold value, C_{A}, said threshold values being input from a user.

11. The method according to claim 8-10, wherein the sensitivity for when alarming a user of an irregular heart rhythm of the user is adjustable by means of adjusting a tolerance number, available to the analyzing unit (118), for said analyzing unit (118) to determine the number of times said frequency irregularity and said amplitude irregularity occurring within said time interval is allowed to occur within a second time interval, before said alarm unit (112) is disabled from alarming the user, said second time interval being longer than said first time interval, wherein said tolerance number is being input from a user.

12. The method according to claim 8-11, wherein the step of calculating (S206), by a frequency unit (114), the frequency of a part of said time-resolved signal comprises
calculating a series of frequency values, each frequency value within said series of frequency values being the inverse of the time period between two adjacent peaks in the time-resolved signal,
and wherein the step of calculating (S208), by an amplitude unit (116), the amplitude of a part of said time-resolved signal comprises
calculating a series of amplitude values, each amplitude value in the series of amplitude values being derived from a single peak in the time-resolved signal,
and wherein the step of analysing (S210), by an analyzing unit (118), said frequency and said amplitude comprises
comparing at least one of the frequency values with one or more other frequency values,
comparing at least one of the amplitude values with one or more other amplitude values.

13. The method according to claim 12, wherein the step of analysing (S210), by an analyzing unit (118), said frequency and said amplitude comprises nonlinear methods comprising a Poincare plot,
wherein analysis is carried out using at least one standard deviation calculated along at least one direction within the plane defined by the Poincare plot.

14. The method according to claim 8-13, wherein the alarm unit induces a tactile sensation in the user, said tactalie sensation pertaining to inform the user that it is time to take an ECG using a second device.

15. The method according to claim 8-14, wherein the alarm unit emits one or more from the list of
a sound alarm pertaining to inform the user that it is time to take an ECG using a second device,
a visual alarm pertaining to inform the user that it is time to take an ECG using a second device.
